# EUROPEAN PATENT SPECIFICATION

(11) **EP 0 870 050 B1**
(45) Date of publication and mention of the grant of the patent: **19.06.2002**
(21) Application number: 96945414.9
(22) Date of filing: 20.12.1996
(51) Int. Cl.: C12N 15/86, C12N 15/47, C12N 7/01, C12N 5/10

(54) **TOXIC-PROTEIN EXPRESSING VIRUSES AND PRODUCER CELL LINES**
TOXISCHES PROTEIN EXPRIMIERENDE VIREN UND PRODUKTIONSZELLINIEN
VIRUS EXPRIMANT DES PROTEINES TOXIQUES, ET LIGNEES CELLULAIRES PRODUCTRICES

(30) Priority: 29.12.1995 US 580556; 29.12.1995 US 580554
(43) Date of publication of application: 14.10.1998
(73) Proprietor: CHIRON CORPORATION, Emeryville California 94608-2916 (US)
(72) Inventor: CHADA, Sunil, Missouri City, Texas 77459 (US); DePOLO, Nicholas, J., Solano Beach, CA 92075 (US); SAUTER, Sybille, Del Mar, CA 92014 (US); ROBBINS, Joan, San Diego, CA 92109 (US); JOLLY, Douglas, J., Leucadia, CA 92924 (US); MANNING, William, C., Redwood City, CA 94061 (US); MURPHY, John, E., Oakland, CA 94618 (US); RALSTON, Robert, O., II, The Woodlands, TX 77381-6317 (US)
(74) Representative: Goldin, Douglas Michael
(86) International application number: US9620587
(87) International publication number: WO9724452

(56) References cited:
- WO-A-87/03905
- WO-A-94/29440
- MANFRED SCHUBERT ET AL.: "Insertion of the Human Immunodeficiency Virus CD4 receptor into the envelope of Vesicular Stomatitis Virus particles" JOURNAL OF VIROLOGY, vol. 66, no. 3, March 1992, pages 1579-1589, XP000644669
- DANIEL S. ORY ET AL.: "A stable human-derived packaging cell line for production of high titer retrovirus/vesicular stomatitis virus G pseudotypes" PROCEEDINGS OF THE NATIONAL ACADEMY OF SCIENCES OF USA, vol. 93, no. 21, 15 October 1996, WASHINGTON US, pages 11400-11406, XP002030515
- BYEONG J. LEE ET AL.: 'Identification of a Selenocysteyl-tRNA-Ser in mammalian cells that recognizes the nonsense codon, UGA.' THE JOURNAL OF BIOLOGICAL CHEMISTRY vol. 264, no. 17, 15 June 1989, pages 9724 - 9727

## Description

### Technical Field

The present invention relates generally to compositions and methods for producing recombinant viruses, and more specifically, to producer cell lines and methods related thereto which allow production of recombinant viruses which encode or contain toxic or fusogenic proteins.

### Background Of The Invention

Since the discovery of DNA in the 1940s and continuing through the most recent era of recombinant DNA technology, substantial research has been undertaken in order to realize the possibility that the course of disease may be affected through interaction with the nucleic acids of living organisms. Most recently, a wide variety of methods have been described for altering or affecting genes, including for example, viral vectors derived from retroviruses, adenoviruses, vaccinia viruses, herpes viruses, and adeno-associated viruses (see Jolly, *Cancer Gene Therapy 1*(1):51-64, 1994).

Recombinant retroviruses and various uses thereof have been described in numerous references including, for example, Mann et al., *Cell 33*:153, 1983, Cane and Mulligan, *Proc*. *Nat'l*. *Acad*. *Sci*. *USA 81*:6349, 1984, Miller et al., *Human Gene Therapy 1*:5-14, 1990, U.S. Patent Nos. 4,405,712, 4,861,719, 4,980,289 and PCT Application Nos. WO 89/02,468, WO 89/05,349 and WO 90/02,806. Briefly, a foreign gene of interest may be incorporated into the retrovirus in place of the normal retroviral RNA. When the retrovirus injects its RNA into a cell, the foreign gene is also introduced into the cell, and may then be integrated into the host's cellular DNA as if it were the retrovirus itself. Expression of this foreign gene within the host results in expression of the foreign protein by the host cell.

Currently, certain retroviral vectors (such as those that have an amphotropic envelope) offer a broad host range, but fail to compare to the host range offered by the VSV-G protein (Marsh and Helenius, *Advances in Virus Research 36*:107-151, 1989). Briefly, Vesicular Stomatitis Virus (VSV) is known to infect various types of tissues including muscle, liver, neuronal, fibroblast and non-replicating hematopoietic cells. VSV-G has an extended tropism compared to amphotropic envelope which enables entry into man, lower primates, rodents, amphibians, fish and insects. A recent report has indicated that VSV G-pseudotyped retroviral vectors can transduce human peripheral hematopoietic stem/ progenitor cells much more efficiently than amphotropic vectors (Kerr et al., *Blood 84* (Suppl. 1):402a, 1994).

However, because of the exquisite fusogenic properties of VSV-G protein, a stable cell line has been very difficult to produce, because of cell fusion throughout the entire culture (Yee *et al*., *Proc*. *Natl*. *Acad*. *Sci. USA 91*:9564-9568, 1994). Some expression of VSV-G has been reported in murine C127 fibroblasts (Florkiewicz *et al*., *J*. *of Cell Biol. 97*:1381-1388, 1983) and MDCK (Roman *et al*., *Experimental Cell Research 175*:376-387, 1988) cells, although levels of cell surface expression were low. WO 94/29440 discloses methods for producing VSV-G pseudotyped retroviral vectors and WO 87/03905 discloses plasmids containing murine CMV promoters.

The present invention provides compositions and methods which allow the production of recombinant viral particles with fusogenic envelopes such as VSV-G, or which express other proteins which are toxic to cells. The present invention provides these, as well as other related advantages.

### Summary of the Invention

Briefly stated, the present invention provides compositions and methods for producing recombinant retroviruses which have toxic and/or fusogenic proteins. For example, within one aspect of the present invention expression vectors are provided comprising a murine CMV IE promoter operably linked to a nucleic acid sequence which encodes a toxic and/or fusogenic protein. Representative examples of fusogenic proteins include Vesicular Stomatitis Virus-G protein, and Rabies virus G protein. Representative examples of toxic proteins include γ-IFN, IL-2, and TNF-β.

Also provided are retroviral vectors which contain one of the above noted expression vectors, packaging cells which comprise a *gag*/*pol* expression cassette and an expression vector as described above, as well as producer cell lines which comprise a *gag*/*pol* expression cassette, an expression vector as described above, and a retroviral vector construct.

Within yet another aspect of the present invention, methods are provided for producing retrovector particles by providing a murine cytomegalovirus in a retroviral producer cell line of the invention. Such a method may further comprise the steps of (a) incubating between 10³ and 10⁵ cells which contain a gag/pol expression cassette and retroviral vector construct in a culture having a surface area greater than 225 cm², under conditions and for a time sufficient to allow the cells to enter the log phase of growth, and (b) introducing an expression vector as described above into the culture, such that retrovector particles may be transiently produced from the cells. Representative examples of methods for introducing the expression vector into the culture of cells such that entry of the expression vector into the cells occurs, include lipofection, electroporation, or transfection utilizing a source of Ca⁺⁺ ions and a source of PO₄⁻⁻.

These and other aspects of the present invention will become evident upon reference to the following detailed description and attached drawings. In addition, various references are set forth below which describe in more detail certain procedures or compositions (*e*.*g*., plasmids, etc.), and are therefore incorporated by reference in their entirety as if each were individually noted for incorporation.

### Brief Description of the Drawings

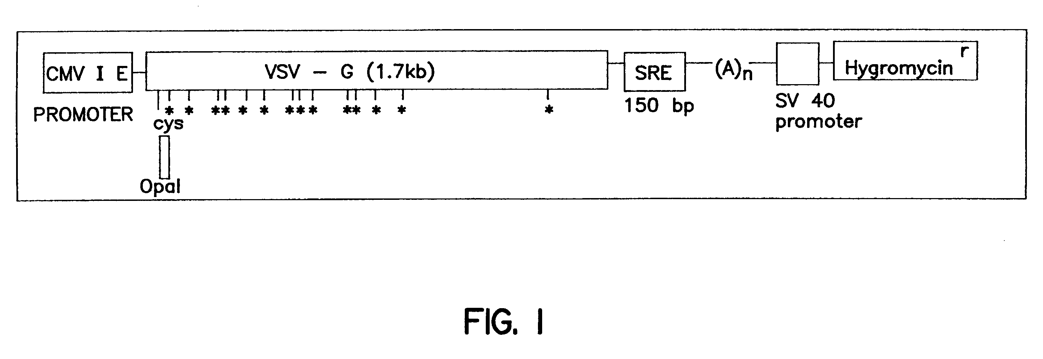

Figure 1 is a schematic illustration of a VSV-G/SRE expression construct.

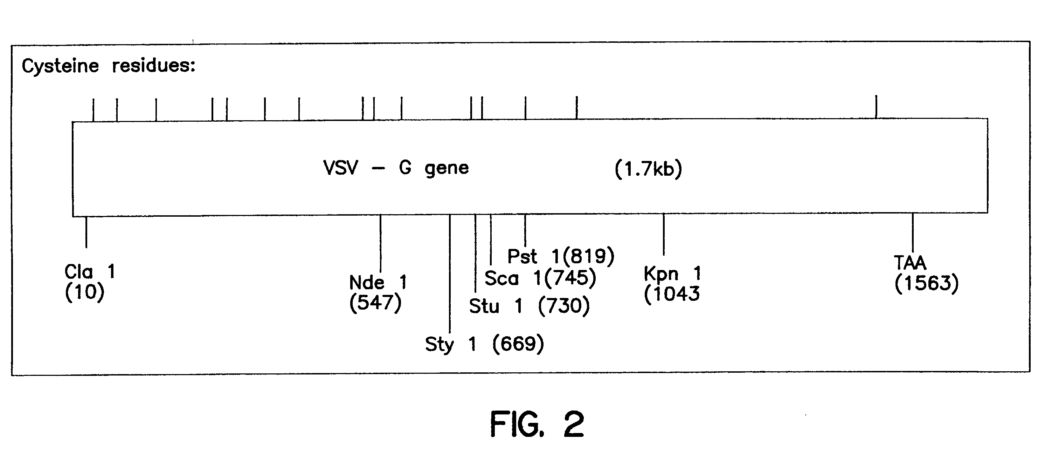

Figure 2 is a schematic illustration of the distribution of cysteine residues in a VSV-G gene.
Figure 3 is a schematic illustration of the HindIII "L" fragment of CMV.
Figure 4A is a Southern blot analysis of three isolates of RM-CMVG. Figure 4B is a schematic illustration of the probe used in the analysis provided in Figure 4A.
Figure 5 is two photographs (200X) of RCMVG and wt MCMV NIH3T3 infected cells.
Figure 6 is a Western blot of VSV-G expressing isolates.
Figure 7 is a Western blot of VSV-G expression isolates.

### Detailed Description of the Invention

Prior to setting forth the invention, it may be helpful to an understanding thereof to set forth definitions of certain terms that will be used hereinafter.

"Expression vector" and "expression cassette" refers to an assembly which is capable of directing the expression of a sequence or gene of interest. The nucleic acid expression vector must include a promoter which, when transcribed, is operably linked to the sequence(s) or gene(s) of interest, as well as a polyadenylation sequence. Within certain embodiments of the invention, both the promoter and the polyadenylation sequence are from a source which is heterologous to the helper elements, *gag*/*pol* and *env*. Within other embodiments of the invention, the expression vectors described herein may be contained within a plasmid construct.

"Retroviral vector," "retroviral vector construct" "RETROVECTOR CONSTRUCT ™" and "recombinant retroviral vector," which generally is a subset of 'expression vectors' as discussed above, refers to a nucleic acid construct which carries, and within certain embodiments, is capable of directing the expression of a nucleic acid molecule of interest. The retroviral vector must include at least one transcriptional promoter/enhancer or locus defining element(s), or other elements which control gene expression by other means such as alternate splicing, nuclear RNA export, post-translational modification of messenger, or post-transcriptional modification of protein. Such retroviral vectors must also include a packaging signal, long terminal repeats (LTRs) or portion thereof, and positive and negative strand primer binding sites appropriate to the retrovirus used (if these are not already present in the retroviral vector). Optionally, the recombinant retroviral vector may also include a signal which directs polyadenylation, selectable markers such as Neo, TK, hygromycin, phleomycin, histidinol, or DHFR, as well as one or more restriction sites and a translation termination sequence. By way of example, such vectors typically include a 5' LTR, a tRNA binding site, a packaging signal, an origin of second strand DNA synthesis, and a 3' LTR or a portion thereof.

"Recombinant retrovirus," "retroviral gene delivery vehicle" and "retroviral vector particle" as utilized within the present invention refers to a retrovirus which carries at least one gene of interest. The retrovirus may also contain a selectable marker. The recombinant retrovirus is capable of reverse transcribing its genetic material into DNA and incorporating this genetic material into a host cell's DNA upon infection.

"Packaging cell" refers to a cell which contains those elements necessary for production of infectious recombinant virus which are lacking in a recombinant viral vector. Typically, such packaging cells contain one or more expression cassettes which are capable of expressing proteins which encode *gag*, *pol* and *env* proteins.

"Producer cell" or "Vector producing cell" refers to a cell which contains all elements necessary for production of recombinant viral vector particles.

"Fusogenic protein" refers to glycoproteins which cause cells within a culture to fuse into a multinucleate syncytia. Representative examples of fusogenic proteins include VSV-G and Rabies G protein.

"Toxic-protein" refers to a protein which exhibits cytotoxic or cytostatic behavior in cells. ). For purposes of the present invention, a protein is deemed to be cytotoxic if, when expressed in a proliferating culture, greater than 20% cell death occurs within a 48 hour time period (as compared to the same culture which does not express the protein). Similarly, a protein is deemed to be cytostatic if, when expressed in a proliferating culture, a decrease in cell doubling time of greater than 20% occurs within a 48 hour time period (as compared to the same culture which does not express the protein). Preferred assays for the measurement of cytotoxicity include the MTT assay which uses an non-radioactive format to quantitate viable cell number in proliferation assays (*e*.*g*., Promega Corp., Madison, WI), or ³H incorporation into nascent DNA. A number of other assay formats however, may likewise be utilized to evaluate various parameters associated with cell toxicity, including for example, cell viability (dye exclusion, cell counting) and cell metabolism (dye reduction) (See Cell Biology, A laboratory Handbook, ed. J.E. Celis, Academic Press for further details and methods. Representative examples of toxic proteins include γ interferon, interleukin-2, TNF α and TNF β.

### A. PREPARATION OF RECOMBINANT RETROVIRAL VECTORS, PACKAGING CELLS, PRODUCER CELLS AND RECOMBINANT RETROVIRUSES

As noted above, the present invention provides recombinant retroviruses which are constructed to carry or express a selected nucleic acid molecule of interest. Retroviral gene delivery vehicles of the present invention may be readily constructed from a wide variety of retroviruses, including for example, B, C, and D type retroviruses as well as spumaviruses and lentiviruses (see RNA Tumor Viruses, Second Edition, Cold Spring Harbor Laboratory, 1985). Such retroviruses may be readily utilized in order to assemble or construct retroviral gene delivery vehicles given the disclosure provided herein, and standard recombinant techniques (*e.g.,* Sambrook et al, *Molecular Cloning: A Laboratory Manual,* 2d ed., Cold Spring Harbor Laboratory Press, 1989; Kunkle, *PNAS 82*:488, 1985).

Representative examples of retroviral gene delivery vehicles that may be utilized within the context of the present invention include, for example, those described in EP 0,415,731; WO 90/07936; WO 94/03622; WO 93/25698; WO 93/25234; U.S. Patent No. 5,219,740; WO 93/11230; WO 93/10218; Vile and Hart, *Cancer Res. 53*:3860-3864, 1993; Vile and Hart, *Cancer Res. 53*:962-967, 1993; Ram et al., *Cancer Res. 53*:83-88, 1993; Takamiya et al., *J*. *Neurosci*. *Res. 33*:493-503, 1992; Baba et al., *J*. *Neurosurg*. *79*:729-735, 1993 (U.S. Patent No. 4,777,127, GB 2,200,651, EP 0,345,242 and WO 91/02805). Particularly preferred recombinant retroviruses include those described in WO 91/02805 and US 95/05789.

Packaging cell lines suitable for use with the above-described vector constructs may be readily prepared (see also WO 92/05266), and utilized to create producer cell lines (also termed vector cell lines or "VCLs") for the production of recombinant vector particles, given the disclosure provided herein.

Within other aspects of the present invention, methods are provided for transiently producing retrovector particles, comprising the general steps of (a) incubating between 10³ and 10⁵ cells which contain a *gag*/*pol* expression cassette and retroviral vector construct in a culture having a surface area greater than 225 cm², under conditions and for a time sufficient to allow the cells to enter log phase of growth, and (b) introducing an expression vector as described above into the culture, such that retrovector particles may be transiently produced from the cells. Representative examples of methods for introducing the expression vector into the culture of cells such that entry of the expression vector into the cells occurs, include lipofection, electroporation, or transfection utilizing a source of Ca⁺⁺ ions and a source of PO₄⁻⁻ (*e.g.,* CaCl and HBS, Promega, Madison WI). Within certain embodiments, the surface area of the culture may be greater than about 1200 cm², or even greater than about 6,000 cm².

Utilizing the methods of the present invention as disclosed herein, packaging cell lines that produce recombinant retroviral particles at titers greater than 10⁶ or 10⁷ cfu/ml (in crude supernatant) may readily be obtained. In addition, it should be noted that such titers are generally obtained from titer assays on HT1080 cells, which produce a three-fold lower titer than titers obtained on murine 3T3 cells.

### B. REGULATION OF GENE EXPRESSION

Within other aspects of the invention, regulation of gene expression may be accomplished by utilizing viral vectors that can replicate in one cell type, but abortively infect another (*i.e.,* are replication competent in their native species and replication incompetent in other species). Such a system may be used for the production of many types of viral vectors, where production of viral proteins is toxic (*e*.*g*., AAV and rep proteins, Adenovirus vectors, Herpes Viral vectors, Sindbis and other alpha viruses).

For example, within one embodiment of the invention, expression vectors are provided comprising a murine CMV IE promoter operably linked to a nucleic acid sequence that encodes a toxic or fusogenic protein. Briefly MCMV is a member of the beta family of Herpes viruses. These viruses contain within their virion a transactivator protein responsible for initiating transcription from the immediate early genes. MCMV abortively infects human cells, *i.e.*, the virus enters the target human cells, some "early gene" expression occurs, but no viral particles are produced. This allows the use of MCMV to express otherwise toxic proteins in a "burst" fashion in human cells, followed by harvest of the product without further production of, and contamination with, MCMV. Therefore the product incorporating the toxic or fusogenic protein can be harvested and used on target cells or tissues without significant MCMV toxicity.

This trans-activation phenomenon may be used to regulate expression of a wide variety of toxic and/or fusogenic genes, such as the VSV-G gene, in at least two distinct methods:
a) The VSV-G gene may be placed under the control of a MCMV immediate early (IE) gene such as ie1 or ie3, by fusing the promoter regions from an IE gene to the VSV-G gene. The modified VSV-G gene is then reintroduced into the MCMV genome by homologous recombination. As VSVg protein is not immediately toxic, and the life cycle of MCMV in mouse cells is two or three days, stocks of MCMV can be produced at conventional titers (10⁷-10⁹ pfu/ml). Recombinant virus stocks are obtained by sequential rounds of plaque purification and may be used to infect human pre-producer cells (cells containing a retroviral vector, but lacking an envelope protein). Because MCMV causes an abortive infection in human cells, VSV-G pseudotyped retroviral vectors will be released from the infected cells with no contamination by MCMV.
b) The VSV-G gene can be placed under the control of the ie gene regulatory sequences from MCMV. This construct can be transfected into a pre-producer cell line (described above). In the absence of the ie transactivators, constitutive transcription from the MCMV IE promoter is very low, resulting in minimal expression of the toxic VSV-G gene protein. If the pre-producer cell is infected with wild type MCMV, the transactivator causes very rapid and strong expression of the VSV-G gene leading to secretion of VSV-G pseudotyped retroviral vectors. Because MCMV causes an abortive infection in human cells, VSV-G pseudotyped retroviral vectors will be released from the infected cells with no contamination by MCMV. In the event that low level MCMV replication does occur in the human pre-producer cells, the MCMV infected cultures may be treated with gancyclovir to prevent synthesis of late MCMV proteins.

Alternatively, the transactivating MCMV need not be functional in terms of replicative capacity and a recombinant MCMV may be engineered which lacks the early or late genes, or both. Such a recombinant MCMV may be propagated in cells stably containing a single copy of the deleted genes (*i*.*e*., early or late) to trans-complement and allow MCMV virus formation.

Although MCMV is provided herein as an example, it should be understood that the present invention is not so limited. In particular, a variety of other viruses, which are replication competent in their native species, and replication incompetent in other species, may likewise be utilized in the present invention. Representative examples include animal viruses such as mouse adapted influenza virus and rhesus rotavirus, as well as other which are set forth below in Table 1.

**TABLE 1**

| **VIRUS** | **PERMISSIVE HOST** | **ABORTIVE INFECTION** |
|---|---|---|
| mCMV | mouse | human |
| fowlpox | birds | human |
| swinepox | pigs | human |
| pseudorabies | pigs, cows, farm animals | human |
| polyoma | mouse | human |
| SV40 | African Green Monkey | human |
| | | |
| temperature-sensitive | cells at permissive | cells at nonpermissive |
| viruses of all kinds | temperature (usually 32C) | temp. (usually 37-39C) |

### C. TOXIC AND FUSOGENIC PROTEINS

As discussed above, a wide variety of either naturally occurring, or synthetically produced toxic or fusogenic proteins may be expressed by the expression vectors and/or viral vectors of the present invention. Representative examples of fusogenic proteins (which mediate a fusogenic state) include VSV-G (Lazarini et al., *J*. *Vir. 45*:773-781, 1983), Rabies virus-G (Gaudin, et al., *J*. *Virol*. *67*:1365-1372, 1993), Semliki Forest virus, Sindbis, and other alpha viruses which have E1 and E2 glycoproteins (Whitt, M. et al., *Virol*. *185*:681, 1991).

Within other aspects of the invention, synthetic chimeras of these glycoproteins may be produced which have altered activities. For example, a synthetic chimera of a rabies extracellular domain fused to VSV-G transmembrane and cytoplasmic domain may be produced, which paradoxically retains infectivity but lacks fusion activity. (Whitt, M. et al., *Virol*. *185*:681, 1991). Additionally, construction of extracellular-transmembrane domain only glycoproteins may be useful for pseudotyping. Representative examples of viruses that may be pseudotyped in this manner include HIV (*Virology 209*(2):696-700, 1995; *J Vir. 69*(3):1984-9, 1995; *AIDS Res Hum Retroviruses 8*(9):1669-77, 1992); SIV (*J*. *Med*. *Primatol 21*(2-3):69-73, 1992); HTLV (*Int*. *J*. *Cancer 60*(4):567-70, 1995; *Int. J*. *Cancer 59*(5):655-60, 1994; *Int*. *J*. *Cancer 59*(3):416-21, 1994; *Int*. *J*. *Cancer 56*(1):100-5, 1994; *J*. *Vir*. *65*(1):162-9, 1991; *Virology 180*(1):420-4, 1991); MuLV (*J*. *Vir*. *67*(8):4712-21, 1993; *J. Vir. 66*(3):1468-75, 1992); FIV (*J*. *Vir*. *67*(7):4142-53, 1993; *P.N.A.S.* *89*(18):8457-61, 1992); RSV (*J*. *Gen*. *Vir. 73* (Pt 11):2995-7, 1992); AKV (*Virology 186*(1)161-6, 1992); Cocal (*J*. *Vir*. *Methods 44*(2-3):287-304, 1993); VSV-G (*Virology 178*(2):373-83, 1990; *J Vir. 5*(3):1202-7, 1991); HSV (*Vaccine 11*(6):675-8, 1993); and LDV (*J*. *Vir. 69*(7):4237-44, 1995).

The present invention may also be useful in the production of recombinant viruses which express toxic proteins. Representative examples of proteins which are toxic to cells include γ-interferon, IL-2, and TNF-β, as well as other toxic molecules (discussed in more detail below) that may be expressed by the recombinant virus, including for example, ricin, abrin, diphtheria toxin, cholera toxin, antiviral protein, tritin, Shigella toxin, and Pseudomonas exotoxin A

### D. OTHER HETEROLOGOUS NUCLEIC ACID MOLECULES

A wide variety of nucleic acid molecules may be carried and/or expressed by the expression vectors or recombinant retroviruses of the present invention. Generally, the nucleic acid molecules which are described herein do not occur naturally in the expression vector or recombinant retrovirus that carries it, and provides some desirable benefit, typically an ability to fight a disease, or other pathogenic agent or condition.

Substances which may be encoded by the nucleic acid molecules described herein include proteins (*e.g.*, antibodies including single chain molecules), immunostimulatory molecules (such as antigens) immunosuppressive molecules, blocking agents, palliatives (such as toxins, antisense ribonucleic acids, ribozymes, enzymes, and other material capable of inhibiting a function of a pathogenic agent) cytokines, various polypeptides or peptide hormones, their agonists or antagonists, where these hormones can be derived from tissues such as the pituitary, hypothalamus, kidney, endothelial cells, liver, pancreas, bone, hemopoetic marrow, and adrenal. Such polypeptides can be used for induction of growth, regression of tissue, suppression of immune responses, apoptosis, gene expression, blocking receptor-ligand interaction, immune responses and can be treatment for certain anemias, diabetes, infections, high blood pressure, abnormal blood chemistry or chemistries (*e*.*g*., elevated blood cholesterol, deficiency of blood clotting factors, elevated LDL with lowered HDL), levels of Alzheimer associated amyloid protein, bone erosion/calcium deposition, and controlling levels of various metabolites such as steroid hormones, purines, and pyrimidines.

Within one aspect of the present invention, methods are provided for administration of an expression vector or a recombinant retrovirus which directs the expression of a palliative. Representative examples of palliatives that act directly to inhibit the growth of cells include toxins such as ricin (Lamb et al., *Eur*. *J*. *Biochem. 148*:265-270, 1985).

Within another aspect of the invention, the expression vector or recombinant retrovirus directs the expression of a substance capable of activating an otherwise inactive precursor into an active inhibitor of a pathogenic agent, or a conditional toxic palliative, which are palliatives that are toxic for the cell expressing the pathogenic condition. As should be evident given the disclosure provided herein, a wide variety of inactive precursors may be converted into active inhibitors of a pathogenic agent. For example, expression vectors or recombinant retroviruses which direct the expression of a gene product (*e*.*g*., a protein) such as Herpes Simplex Virus Thymidine Kinase (HSVTK) or Varicella Zoster Virus Thymidine Kinase (VZVTK) which assists in metabolizing antiviral nucleoside analogues to their active form are therefore useful in activating nucleoside analogue precursors (*e.g.*, AZT or ddC) into their active form. AZT or ddI therapy will thereby be more effective, allowing lower doses, less generalized toxicity, and higher potency against productive infection. Additional nucleoside analogues whose nucleotide triphosphate forms show selectivity for retroviral reverse transcriptase but, as a result of the substrate specificity of cellular nucleoside and nucleotide kinases are not phosphorylated, will be made more efficacious.

In yet another aspect, expression vectors and recombinant retroviruses are provided which have a therapeutic effect by encoding one or more ribozymes (RNA enzymes) (Haseloff and Gerlach, *Nature 334*:585, 1989) which will cleave, and hence inactivate, RNA molecules corresponding to a pathogenic function (*see also,* Foster and Symons, *Cell 48*: 211-220, 1987; Haseloff and Gerlach, *Nature 328*: 596-600, 1988; Ruffner et al., *Biochem 29*: 10,695-10,702, 1990; U.S. Patent Nos. 5,254,678 and 4,987,071; and PCT Publication No. WO93/23569). In another aspect, expression vectors and recombinant retroviruses are provided comprising a biologically active nucleic acid molecule that is an antisense sequence (an antisense sequence may also be encoded by a nucleic acid sequence and then produced within a host cell via transcription). In preferred embodiments, the antisense sequence is selected from the group consisting of sequences which encode influenza virus, HIV, HSV, HPV, CMV, and HBV. The antisense sequence may also be an antisense RNA complementary to RNA sequences necessary for pathogenicity. Alternatively, the biologically active nucleic acid molecule may be a sense RNA (or DNA) complementary to RNA sequences necessary for pathogenicity.

Still further aspects of the present invention relate to recombinant retroviruses capable of immunostimulation. Briefly, the ability to recognize and defend against foreign pathogens is essential to the function of the immune system. In particular, the immune system must be capable of distinguishing "self" from "nonself" (*i.e.,* foreign), so that the defensive mechanisms of the host are directed toward invading entities instead of against host tissues. Cytolytic T lymphocytes (CTLs) are typically induced, or stimulated, by the display of a cell surface recognition structure, such as a processed, pathogen-specific peptide, in conjunction with a MHC class I or class II cell surface protein.

In one embodiment, the invention provides methods for stimulating a specific immune response and inhibiting viral spread by using an expression vector or recombinant retroviruses that direct the expression of an antigen or modified form thereof in susceptible target cells, wherein the antigen is capable of either (1) initiating an immune response to the viral antigen or (2) preventing the viral spread by occupying cellular receptors required for viral interactions.

In another aspect of the invention, the expression vectors or recombinant retroviruses of the present invention may be constructed to express "immunomodulatory factors." Immunomodulatory factors refer to factors that, when manufactured by one or more of the cells involved in an immune response, or, which when added exogenously to the cells, causes the immune response to be different in quality or potency from that which would have occurred in the absence of the factor. The quality or potency of a response may be measured by a variety of assays known to one of skill in the art including, for example, *in vitro* assays which measure cellular proliferation (*e*.*g*., ³H thymidine uptake), and *in vitro* cytotoxic assays (*e.g.,* which measure ⁵¹Cr release) (*see,* Warner et al., *AIDS Res*. *and Human Retroviruses 7*:645-655, 1991). Immunomodulatory factors may be active both *in vivo* and *ex vivo*.

Representative examples of such immunomodulatory factors include cytokines or lymhokines, interferons (*e*.*g*., γ-IFN), tumor necrosis factors (TNFs) (Jayaraman et al., *J. Immunology 144*:942-951, 1990), CD3 (Krissanen et al., *Immunogenetics 26*:258-266, 1987), ICAM-1 (Altman et al., *Nature 338*:512-514, 1989; Simmons et al., *Nature 331*:624-627, 1988), ICAM-2, LFA-1, LFA-3 (Wallner et al., *J*. *Exp*. *Med*. *166*(4):923-932, 1987), MHC class I molecules, MHC class II molecules, B7.1-.3, β₂-microglobulin (Parnes et al., *PNAS 78*:2253-2257, 1981), chaperones such as calnexin and MHC linked transporter proteins or analogs thereof (Powis et al., *Nature 354*:528-531, 1991).

The present invention also includes expression vectors and recombinant retroviruses which encode immunogenic portions of desired antigens including, for example, viral, bacterial or parasite antigens. Representative examples include hepatitis Band C viral antigens (*e.g.*, WO 93/15207), feline leukemia virus and/or immonudeficiency virus antigens (*e.g.*, WO 94/06921). Still other examples include expression vectors or recombinant retroviruses which direct the expression of a non-tumorigenic, altered genes such as the ras (ras*) gene and the p53 gene (*see* WO 93/10814).

### E. CELL CULTURE CONCENTRATION AND PURIFICATION OF RECOMBINANT RETROVIRAL PARTICLES

As noted above, the present invention provides recombinant retroviral preparations suitable for administration to humans and other warm-blooded animals. In particular, a wide variety of methods may be utilized in order to produce recombinant retroviruses suitable for administration, including for example, the use of fermenters or bioreactors, roller bottles, cell hotels or cell factories, and hollow fiber culture.

Briefly, for bioreactors or fermenters, cells are preferably grown on microcarriers (*i*.*e*., Cytodex 1 or Cytodex 2; Pharmacia, Piscataway, N.J. at concentrations ranging from 3 to 15 g/L microcarrier. For roller bottles, suitable conditions include those described above for bioreactors, with the exception that microcarrier beads are generally not preferred. Representative examples of such methods, as well as other methods such as cell holds or cell factories and hollow linker culture, are described within WO 96/0926.

Subsequent to culturing, a wide variety of methods may be utilized for increasing viral concentration and purity, including for example, precipitation of recombinant retroviruses with ammonium sulfate, polyethylene glycol ("PEG") concentration, concentration by centrifugation (either with or without gradients such as PERCOLL, or "cushions" such as sucrose, use of concentration filters (*e.g.*, Amicon filtration), and 2-phase separations. Representative examples of concentrating and purifying and recombinant retroviral particles are described within WO 96/0926.

### F. ADMINISTRATION

As noted above, high titer recombinant retroviral particles of the present invention may be administered to a wide variety of locations including, for example, into sites such as the cerebral spinal fluid, bone marrow, joints, arterial endothelial cells, rectum, buccal/sublingual, vagina, the lymph system, to an organ selected from the group consisting of lung, liver, spleen, skin, blood and brain, or to a site selected from the group consisting of tumors and interstitial spaces. Within other embodiments, the recombinant retrovirus may be administered intraocularly, intranasally, sublinually, orally, topically, intravesically, intrathecally, topically, intravenously, intraperitoneally, intracranially, intramuscularly, or subcutaneously. Other representative routes of administration include gastroscopy, ECRP and colonoscopy, which do not require full operating procedures and hospitalization, but may require the presence of medical personnel.

The following examples are offered by way of illustration, and not by way of limitation.

### EXAMPLES

### EXAMPLE 1

### PREPARATION OF RETROVIRAL VECTOR BACKBONES

Retroviral backbones suitable for use with the present invention may be readily prepared by one of skill in the art. Representative examples of such backbones, such as KT-1 and KT-3, are described in WO 91/02805 and WO 95/05789.

### EXAMPLE 2

### RECOVERY OF THE VSV-G GENE FROM THE PLASMID MLP-G

The VSV-G gene (Lazarini et al., *J*. *Vir*. *45*:773-781, 1983; *see also*, Sequence I.D. Nos. 1 and 2) is isolated from a plasmid containing VSV-G (*e*.*g*., pMLP-G, which has the adenoviral major late promoter and the tri-partite leader sequence (Kaufman, *P*.*N*.*A*.*S*. *82*:689-693, 1985; Ballay et al., in *Hepadna Viruses,* W. Robinson, K. Koike and H. Will (eds.), pp. 173-187, Liss, New York, 1987; *see also* WO 92/05266) as a 1.6 kb PCR product using primers #5 and #2, which also introduce a *Sac* II site at the 5' and an *Eco*R I site at the 3' end, respectively.

Primer-2: (Sequence ID No. 3)
5'- GCC GAA TTC AAT TGA GGC CTC TTT G- 3' (25-mer)

Primer-5: (Sequence ID No. 4)
5'- GAT CCG CGG AGA ATC GAT CTG TTT CCT TGA CAC TAT
GAA GTG CCT TTT G- 3' (49-mer)

The PCR product is cloned into the TA cloning vector pCR™II (INVITROGEN CORP., San Diego, CA), and checked for the correct sequence via partial sequencing and correct orientation of the VSV-G gene. The VSV-G gene is then recovered from the TA cloning vector and ligated into the eukaryotic expression vector pcDNAIII (INVITROGEN). The PCR clone is then checked for functionality by co-transfection of 293(2-3) cells with the VSV-G expressing pcDNAIII and pCB/β-gal at a ratio of 1:1 using the profectin kit from PROMEGA. As a positive control, pMLP-G is cotransfected with the pCB/β-gal plasmid in parallel. After the cotransfection, fresh media is added to the cells, supernatant collected and sterile-filtered (0.45 mm) 24 hours later. HT1080 target cells are then transduced with both supernatants at various dilutions as follows.

On day one, three wells of a 6-well plate are seeded with 2x10⁵ HT1080 in 2 ml media (DMEM high glucose with 10% FBS, nonessential amino acids and sodium pyruvate) per well. Cells are incubated for 24 hours at 37°C and 10% CO₂ before transduction the next day.

On day two the media is removed, and one ml media containing 8 µg polybrene/ml final is added per well. Cells are incubated for 30 minutes at 37°C and 10% CO₂. The supernatant is diluted by gentle pipetting of vector containing media to an approximate concentration of 5 cfu/ml. Five, 20 and 50 ul of the diluted supernatant is added into wells 1 to 3, respectively, the media swirled and incubated at 37°C and 10% CO₂ for 24 hours.

On day three one ml of media is added per well and cells are incubated at 37°C and 10% CO₂ for 24 hours.

On day four an X-gal staining procedure on the transduced cells is carried out. The total number of blue cells is counted per well and the titer determined. Briefly, the media is drained, and 0.5 ml of fixing solution (PBS with 2% (v/v) formaldehyde and 0.2% (v/v) glutaraldehyde) is added per well. The cells are incubated for 5 minutes at room temperature, the fixing solution drained and the cells washed with 2 ml PBS per well. The wells are drained and 0.5 ml of freshly mixed X-gal stain (PBS with 5 mM potassium ferrocyanide, 5 mM potassium ferricyanide, 2 mM MgCl₂, and 1 mg/ml X-gal) is added per well. Cells are incubated from 2 hours to overnight at 37°C and blue cells counted.

If the titer between the pMLP-G and the PCR-based VSV-G gene product in the expression vector is comparable, the PCR-based VSV-G gene is sequenced by Lark Sequencing Technologies and compared to the VSV-G sequence in pMLP-G. If the titer is much lower or zero with the VSV-G gene retrieved by PCR, other plasmid clones with VSV-G ligated onto PCR II are screened.

The functional VSV-G gene can be recovered from either the prokaryotic TA cloning vector pCR™II or from the eukaryotic expression vector pcDNAIII.

### EXAMPLE 3 (REFERENCE EXAMPLE)

### USE OF THE TETRACYCLINE-INDUCIBLE PROMOTER SYSTEM TO PRODUCE A STABLE, VSV-G EXPRESSING PACKAGING CELL LINE

### A. Introduction

As described in more detail below, the tetracycline transactivator (tTA) is introduced into the 293(2-3) PCL and constitutively expressed. A second transfection of the 293(2-3)tTA cell line introduces the tet operator sequences with the VSV-G gene fused behind the tet operator sequences.

### B. Plasmid Preparations

Plasmid preparations of each of the following plasmids are carried out using the QIAGEN plasmid kits (QIAGEN Inc., Chatsworth, CA) essentially as described by the manufacturer. Briefly, the plasmids are electroporated into *E*. *coli* DH 12S (Life Technologies, Inc., Gaithersburg, MD), and cultures are grown in LB broth complemented with ampicillin at 100 µg/ml final concentration for all the plasmids except for pMLP-G, which requires tetracycline at 5 µg/ml final concentration. Electroporated bacteria are plated as single colonies on LB agar plates containing the respective antibiotics. Single colonies are selected, mini plasmid preparations carried out and a characteristic restriction enzyme digest performed to verify identity of each plasmid. Once a single clone is identified, glycerol cultures (1:1 dilution of 40% glycerol and bacterial suspension) are stored at -80°C. The table below lists the available plasmids, describes the coding regions and the source of the plasmids

| PLASMIDS FOR THE PRODUCTION OF A STABLE, TETRACYCLINE-INDUCIBLE VSV-G PCL | | |
|---|---|---|
| Name | Coding Regions | Reference |
| pUHD 15-20 | tTA under HSV-Tk promoter | *see, e*.*g*., U.S. Patent No. 5,464,758 |
| | | |
| pUHD 15-1 | tTA under hCMV promoter/enhancer | U.S. Patent No. 5,464,758; Gossen M. and Bujard H., *PNAS 89*:5547-5551 (1992) |
| | | |
| pUHD 10-3 | hCMV promoter/enhancer with heptamerized upstream tet-operators as described in ref. above, followed by a multiple cloning site | Gossen and Bujard, *PNAS 89*:5547-5551 (1992) |
| | | |
| pMLP-G | VSV-G gene under the MLP promoter | Lazzarini et al., *J*. *Virol*. *45*:773-781(1983) |
| | | |
| pT3/T7-Luc | Luciferase expression vector | Clontech (Clontech Inc., Palo Alto, CA) |
| | | |
| pUT 507 | Phleomycin/Zeocin resistance plasmid | Tiraby et al., *Somatic Cell and Molecular Genetics 14*:243-252 (1988) |
| | | |
| pHIS | Histidinol marker plasmid | (see below) |
| | | |
| pCB/β-gal | β-gal coding plasmid | Irwin et al., *J*. *Vir*. *68*(8): 5036-5044, 1994 |

The eukaryotic expression vector, pREP8 (Invitrogen, San Diego, CA), which contains the histidinol resistance gene, as well as the Epstein Barr virus origin of replication and EBNA, is digested with *Eco*RI and a 5.8 Kb fragment isolated, religated and transfected into *E*. *coli.* This vector, pHIS, contains only the plasmid origin of replication, ampicillin resistance gene and the histidinol resistance gene with the SV40 promoter and polyadenylation sequences.

### C. Construction of the Luciferase Reporter Plasmid pUHD 10-3/luc

Tetracycline transactivator function is first examined in a transient transfection assay using a tTA-controlled luciferase reporter unit. Both regulator plasmids, pUHD 15-20 as well as pUHD 15-1 are analyzed in the transient assays to determine which plasmid gives a better system in terms of leakiness and level of activation after tetracycline withdrawal. The cotransfection experiments are carried out in 293(2-3) cells and D-17 gag/pol cells, as well as HeLa cells as a positive control.

### 1. Cloning of the Luciferase Gene into pUHD 10-3.

Plasmid pUHD 10-3 is linearized with *Eco*R I (New England Biolabs, Inc., Beverly, MA), the 5' overhang filled in with nucleotides and the Klenow fragment in the restriction enzyme buffer. The vector is then treated with PCI (phenol:chloroform:isoamylalcohol, Amresco Inc., Solon, Ohio) to remove proteins, and ethanol-precipitated. *Hind* III linkers are ligated to the plasmid, the plasmid restricted with *Hind* III and dephosphorylated with CIP (Calf Intestinal Phosphatase). After dephosphorylation, the vector is PCI-extracted and gel-purified using the Qiaex gel purification kit from Qiagen, essentially according to manufacturer's procedures.

The luciferase gene is isolated from pT3/T7-Luc (Clontech) as a 1.9 kb fragment after a *Hind* III digest (New England Biolabs), the two fragments purified over an agarose gel to separate the 1.9 kb luciferase-coding fragment from the 3.15 kb vector and the luciferase gene ligated into the HindIII restricted pUHD 10-3 vector to produce pUHD 10-3/luc.

An aliquot of the ligation is electroporated into *E*. *coli* DH 12S (Life Technologies), the cultures grown in LB broth for one hour at 37°C, an aliquot plated onto LB agar plates complemented with ampicillin at 100 µg/ml final concentration and incubated at 37°C overnight. Single colonies are selected, grown in 2 ml LB broth supplemented with ampicillin to 100 µg/ml final concentration overnight and a plasmid mini preparation using the Qiagen miniprep kit (Qiagen Inc., Chatsworth, CA) according to manufacturer's instructions.

In order to identify clones carrying the luciferase gene, a restriction enzyme double digest with *Sal* I and *Sac* I (New England Laboratories) or a single digest with *Hind* III (New England Laboratories) is carried out. The electrophoresis of the digest on a 1% analytical agarose gel shows the 3.15 kb linearized vector and a 1.9 kb fragment for the luciferase gene. Clones with both fragments are analyzed further with respect to the orientation of the luciferase gene in the vector. A *Xba* I digest (New England Laboratories) is performed and the electrophoresed fragments examined on a 1% agarose gel. The fragment pattern for the correct orientation is 0.14, 1.76 and 3.15 kb and for the inverse orientation 0.14 and 5.0 kb.

A plasmid preparation of a clone with the correct luciferase orientation (pUHD 10-3/luc) is carried out using the Qiagen plasmid kit.

### 2. Transient Expression of Luciferase in 293(2-3), D-17 gag/pol and HeLa Cells.

293(2-3) and D-17 packaging cells (WO 92/05266) (as well as HeLa cells as a positive control), are cotransfected with pUHD 10-3/luc and either pUHD 15-20 or pUHD 15-1 at ratios of 1:20, 1:50 and 1:100. The plasmids are introduced via calcium phosphate precipitation using the profection kit from Promega (Promega Corp., Madison, WI) as recommended by manufacturer. Two tissue culture dishes of each of the various combinations of plasmids, ratios and cell lines are set up. Tetracycline, anhydrocycline or deoxycycline is added to one dish but not to the other.

One of the tetracyclines is added at 1 µg/ml final concentration several hours before the cotransfection. The cells of both dishes per combination are analyzed 24 hours post transfection for luciferase activity.

### 3. Analysis of Luciferase Activity.

The luciferase assay is carried out according to manufacturer's instructions (Promega). The tTA regulator plasmid (pUHD 15-20 or pUHD 15-1) that gives the lowest basal activity and the highest degree of activation of luciferase under tetracycline-free culture is utilized in order to establish a stable VSV-G producing 293(2-3) and or D-17 cell line.

### 4. Construction of a Stable 293(2-3)tTA and D-17 gag/pol tTA Cell Lines

The regulatory system in 293(2-3) and D-17 *gag*/*pol* cells is established in two steps. First, 293(2-3) and D-17 *gag*/*pol* cells are cotransfected with a tTA regulator plasmid and pUT 507 at a ratio of 10:1. The plasmids are introduced via calcium phosphate precipitation using the profection kit from PROMEGA. The cotransfection is carried out as recommended by manufacturer. Two days after the cotransfection the cells are selected with zeocin for 10 days until all the non-resistant cells are dead. The cells are then taken off the selection and a limiting dilution is carried out in several 96-well plates such that one third of the wells shows growth. Each clone is transiently transfected with pUHD 10-3/luc and screened for luciferase activity. Clones with the high levels of luciferase activity and low basal levels in the presence of tetracycline are used to introduce the VSV-G gene cloned into pUHD 10-3. The cells are then taken off the selection and a limiting dilution is carried out in several 96-well plates such that one third of the wells shows growth. Each clone is transiently transfected with pUHD 10-3/luc and screened for luciferase activity. Clones with the high levels of luciferase activity and low basal levels in the presence of tetracycline are used to introduce the VSV-G gene cloned into pUHD 10-3.

### D. Construction of the VSV-G Expression Plasmid pUHD 10-3/VSV-G

The VSV-G gene is recovered from pCR™II TA cloning vector with an Eco-RI-SacII double digest. The 1.6 kb VSV-G gene is isolated and purified by electrophoresis over a 1% agarose gel using the QIAEX kit as described above. The plasmid pUHD 10-3 is restricted with SacII and EcoRI and the VSV-G gene ligated into the backbone.

An aliquot of the ligation is electroporated into *E*. *coli* DH 12S (Life Technologies), the cultures grown in LB broth for one hour at 37°C, an aliquot plated onto LB agar plates complemented with ampicillin to 100 µg/ml final concentration and incubated at 37°C overnight. Single colonies are picked, grown in 2 ml LB broth complemented with ampicillin to 100 µg/ml final concentration overnight and a plasmid mini preparation carried out as described above.

In order to identify clones carrying the VSV-G gene, a restriction enzyme double digest with *Sac* II and *Xba* I (New England Laboratories) is carried out. The electrophoresis of the digest on a 1% analytical agarose gel shows the 3.15 kb linearized vector and a 1.6 kb fragment for the VSV-G gene.

A plasmid preparation of a clone with the correct VSV-G orientation (pUHD 10-3/VSV-G) is carried out using the Qiagen plasmid kit.

### E. Construction of a Stable 293(2-3)tTA/VSV-G and/or D-17 gag/pol tTA/VSV-G Cell Lines

The VSV-G gene is introduced into the 293(2-3)tTA and/or D-17 tTA-VSV-G clone by cotransfection of pUHD 10-3/VSV-G and pUT 507, followed by selection with phleomycin or zeocin.

Both plasmids are introduced into 293(2-3)tTA and D-17 *gag*/*pol* cells at a ratio of 10:1 via calcium phosphate precipitation using the profection kit from Promega as recommended by manufacturer. From the time of cotransfection, the cells are kept in tetracycline containing media (1 µg/ml final concentration). Two days after the cotransfection the cells are selected with histidinol for 10 days until all the non-resistant cells are dead. The cells are then taken off the selection and a limiting dilution is carried out in several 96-well plates such that one third of the wells shows growth. Each clone is split into two tissue culture dishes, one dish containing media without tetracycline to induce the production of VSV-G. One day later, the cells in media without tetracycline are transfected with the β-gal gene (from pCB/β-gal) via calcium phosphate transfection in order to determine the titering potential of each of the PCL clones. Two days after the transfection, the supernatant is harvested and HT1080 target cells transduced with the viral particles carrying the β-gal gene as follows.

On day one, per supernatant three wells of a 6-well plate are seeded with 2 x 10⁵ HT1080 in 2 ml media (DMEM high glucose with 10% FBS, nonessential amino acids and sodium pyruvate) per well. Cells are incubated exactly for 24 hours at 37°C and 10% CO₂ before transduction the next day.

On day two the media is removed, and one ml media containing 8 mg polybrene/ml is added per well. Cells are incubated for 30 minutes at 37°C and 10% CO₂. Dilute the supernatant by gentle pipetting in media to an approximate concentration of 5 CFU/ml. Add 5, 20 and 50 ml of the diluted supernatant into wells 1 to 3, respectively, swirl media and incubate at 37°C and 10% CO₂ for 24 hours.

On day three one ml of media is added per well and cells incubated at 37°C and 10% CO₂ for 24 hours.

On day four an X-gal staining procedure on the transduced cells is carried out. The total number of blue cells is counted per well and the titer determined.

For the X-gal staining the media is drained, 0.5 ml of fixing solution (PBS with 2% (v/v) formaldehyde and 0.2% (v/v) glutaraldehyde) added per well of a 12-well plate, the cells incubated for 5 minutes at room temperature, the fixing solution drained, cells washed with 2 ml PBS per well, wells drained and 0.5 ml of freshly mixed X-gal stain (PBS with 5 mM potassium ferrocyanide, 5 mM potassium ferricyanide, 2 mM MgCl₂, and 1 mg/ml X-gal) added per well, cells incubated from 2 hours to overnight at 37°C and blue cells counted.

Once the titering potential is determined, four clones with the highest capacity to produce infectious viral particles are kept in culture for several weeks in media containing tetracycline in order to determine the basal levels of VSV-G expression. Only clones that do not show effects of VSV-G toxicity are useful as stable VSV-G PCLs.

### EXAMPLE 4 (REFERENCE EXAMPLE)

### USE OF IPTG/TEMPERATURE-INDUCIBLE LAP PROMOTER SYSTEMS TO PRODUCE A STABLE, VSV-G EXPRESSING PACKAGING CELL LINE

As described in more detail below, each of the LAPs driven by a strong promoter is introduced into the 293(2-3) packaging cell line and constitutively expressed. A second transfection of the 293(2-3)LAP cell line introduces the LAP binding domain (lac operator sequences) driven by a weak promoter with the VSV-G gene fused behind the lac operator sequences.

### A. Description of the LAP267 and LAP348 Promoter Systems

LAP348: This promoter system is negatively regulated by IPTG, therefore the expression of the VSV-G envelope is repressed after addition of IPTG.

LAP267: In contrast, LAP267 allows expression of the VSV-G envelope after addition of IPTG and/or down-shift of temperature to 32°C. LAP267 contains an insertion of the transcriptional activation domain of HSV VP 16 within the inducer-binding and dimerization domain of the lac repressor protein which makes the LAP267 protein temperature-sensitive. IPTG has no effect on LAP267 activity at the permissive temperature (32°C). LAP267 activity is rescued by IPTG at 39.5°C.

### B. Plasmid Preparations

Plasmid preparations of each of the following plasmids are carried out using the Qiagen plasmid kits (Qiagen Inc., Chatsworth, CA) according to manufacturer's suggestions. Briefly, the plasmids are electroporated into *E*. *coli* DH 12S (Life Technologies, Inc., Gaithersburg, MD), the cultures grown in LB broth complemented with ampicillin at 100 µg/ml final concentration for all the plasmids except for pMLP-G which needs tetracycline at.5 µg/ml final concentration. Electroporated bacteria are plated as single colonies on LB agar plates containing the respective antibiotic, single colonies picked, mini plasmid preparations carried out and a characteristic restriction enzyme digest performed to verify identity of each plasmid. Once a single clone is identified, glycerol cultures (1:1 dilution of 40% glycerol and bacterial suspension) are stored at -80°C. The table below lists the available plasmids, describes the coding regions and the source of the plasmids.

| PLASMIDS FOR THE PRODUCTION OF A STABLE, IPTG-INDUCIBLE VSV-G PCL | | |
|---|---|---|
| Name | Coding Regions | Reference |
| pCMV267 | LAP267 under minimal CMV promoter | *PNAS 88*:5072-5076, 1991 |
| | | |
| pHCMVLAP348 | LAP348 under minimal CMV promoter | *Molec*. *Cell. Biol*. *10*:3343-3356, 1990 |
| | | |
| pL7CAT | One set of tandem A and B lac operator repeats followed by SV40 promoter and CAT gene | *Molec*. *Cell. Biol*. *10*:3343-3356, 1990 |
| | | |
| pL14CAT | Two sets of tandem A and B lac operator repeats followed by SV40 promoter and CAT gene | *Molec*. *Cell. Biol*. *10*:3343-3356, 1990 |
| | | |
| pMLP-G | VSV-G gene under the MLP promoter | *J*. *Vir*. *45*:773-781, 1983 |
| | | |
| pT3/T7-Luc | Luciferase expression vector | Clontech (Clontech Inc., Palo Alto, CA) |
| | | |
| pUT 507 | Phleomycin/Zeocin resistance plasmid | *Somatic Cell and Molecular Genetics 14*:243-252, 1988 |
| | | |
| pHIS | Histidinol marker plasmid | (see above) |
| | | |
| pCB/β-gal | β-gal coding plasmid | Irwin et al., *J. Vir*. *68*(8):5036-5044, 1994 |

### C. Construction of the Luciferase Reporter Plasmids pL7/luc and pL14/luc

The LAP functions are first examined in a transient transfection assay using a LAP267 or LAP348-controlled luciferase reporter unit. Combinations of both regulator plasmids, pCMV267 and pHCMVLAP348, together with either of the two reporter plasmids pL7/luc and pL14/luc are analyzed in the transient assays to determine which plasmid combination gives a better system in terms of leakiness and level of activation after IPTG/temperature induction. The cotransfection experiments are carried out in 293(2-3) and D-17 *gag*/*pol* cells, as well as HeLa cells as a positive control.

### D. Cloning of the Luciferase Gene into pL7CAT and pL14CAT

The *Stu* I-*Bal* I fragment from pL7CAT and pL 14CAT coding for the CAT gene is deleted using a double digest of each of the two reporter plasmids *with Stu* 1 and *Bal* I. The two resulting blunt-ended fragments for each plasmid are separated in a 1% agarose gel and the pL7 and pL14 backbone are cut out, gel purified using the Qiaex gel purification kit from Qiagen according to manufacturer's procedures. This step eliminates the CAT gene from the pL7CAT and pL 14CAT plasmids. HindIII linkers are ligated to each of the backbones, the backbones restricted with HindIII and treated with calf intestinal phosphatase to decrease the chance of religation. The luciferase gene is isolated from the pT3/T7-Luc plasmid by HindIII digest, the 1.9 kb luciferase gene isolated and purified over a 1% agarose gel as described above and the luciferase fragment ligated into the HindIII sites of each of the plasmid backbones.

An aliquot of the ligation is electroporated into *E. coli* DH 12S (Life Technologies), the cultures grown in LB broth for one hour at 37°C, an aliquot plated onto LB agar plates complemented with ampicillin at 100 µg/ml final concentration and incubated at 37°C overnight. Single colonies are picked, grown in 2 ml LB broth supplemented with ampicillin to 100 µg/ml final concentration overnight and a plasmid mini preparation using the Qiagen miniprep kit (Qiagen Inc., Chatsworth, CA) according to manufacturer's instructions.

In order to identify clones carrying the luciferase gene, an analytical restriction enzyme digest is carried out. A plasmid preparation of one clone each with the correct luciferase orientation (pL7/luc and pL14/luc) is carried out using the Qiagen plasmid kit.

### E. Transient Expression of Luciferase in 293(2-3) D-17 gag/pol and Hela Cells

293(2-3), D-17 *gag*/*pol* and HeLa cells as positive control are cotransfected with either pL7/luc or pL14/luc and either pCMV267 or pHCMVLAP348 at ratios of 1:20, 1:50 and 1:100. The plasmids are introduced via calcium phosphate precipitation using the profection kit from Promega (Promega Corp., Madison, WI) as recommended by manufacturer. Two tissue culture dishes of each of the various combinations of plasmids, ratios and cell lines are set up. One of the two dishes is induced with IPTG at 5 mM and/or temperature shift for the LAP267 containing combinations and IPTG withdrawal for the LAP348 containing combinations.

Cells are induced several hours before the cotransfection. The cells of both dishes per combination are analyzed 24 hour post transfection for luciferase activity.

### F. Analysis of Luciferase Activity

The luciferase assay is carried out according to manufacturer's instructions (Promega).

The LAP regulator plasmid (pCMV267 or pHCMVLAP348) that gives the lowest basal activity and the highest degree of activation of luciferase under non-inducing culture conditions is utilized in order to establish a stable VSV-G producing 293(2-3) and/or D-17 *gag*/*pol* cell line.

### G. Construction of a Stable 293(2-3)LAP267, D-17 gag/pol LAP267, D-17 gag/pol LAP348 and/or 293(2-3)LAP348 Cell Line

The regulatory system in 293(2-3) and D-17 *gag*/*pol* cells is established in two steps. First, 293(2-3) and D-17 *gag*/*pol* cells are cotransfected with a LAP regulator plasmid and pUT507 at a ratio of 10:1. The plasmids are introduced via calcium phosphate precipitation using the profection kit from Promega, essentially according to the manufacturer's instructions. The cotransfection is carried out as recommended by manufacturer. Two days after the cotransfection the cells are selected with G418 at 0.8 mg/ml for 10 days until all the non-resistant cells are dead. The cells are then taken off the selection and a limiting dilution is carried out in several 96-well plates such that one third of the wells shows growth. Each clone is transiently transfected with pL7/luc or pL14/luc and screened for luciferase activity. Clones with high levels of luciferase activity and low basal levels under non-inducing culture conditions are used to introduce the VSV-G gene cloned into pL7 or pL14.

### H. Construction of the VSV-G Expression Plasmids pL7/VSV-G and pL14/VSV-G

The VSV-G gene is recovered from the pCR™II TA cloning vector with an EcoRI digest. The 1.6 kb VSV-G gene is isolated and purified by electrophoresis over a 1% agarose gel using the QIAEX kit as described above.

The plasmids pL7CAT and pL14CAT are digested with *Stu*I and *Bal*I which deleted the CAT gene. *Eco*RI linkers are ligated to the blunt ends, the backbones restricted with *Eco*RI and treated with calf intestinal phosphatase. Then the 1.6 kb VSV-G is ligated into the pL7 and pL14 backbones.

An aliquot of the ligation is electroporated into *E*. *coli* DH 12S (LIFE TECHNOLOGIES), the cultures grown in LB broth for one hour at 37°C, an aliquot plated onto LB agar plates complemented with ampicillin to 100 µg/ml final concentration and incubated at 37°C overnight. Single colonies are picked, grown in 2 ml LB broth complemented with ampicillin to 100 µg/ml final concentration overnight and a plasmid mini preparation carried out as described above.

An analytical restriction enzyme digest is carried out for several clones of each of the ligations in order to identify the correct ligation. A plasmid preparation of a clone for each ligation with the correct VSV-G orientation (pL7/VSV-G and pL14/VSV-G) is carried out using the Qiagen plasmid kit.

### I. Construction of a Stable 293(2-3)LAP267/VSV-G D-17 gag/pol LAP267/VSrG and/or 293(2-3)LAP348/VSV-G, D-17 gag/pol LAP 348/VSV-G Cell Lines

The VSV-G gene is introduced into the 293(2-3)LAP and D-17 *gag*/*pol* LAP clones in a cotransfection of pL7/VSV-G or pL14/VSV-G and pHIS for selection with histidinol.

Both plasmids are introduced into 293(2-3)LAP and D-17 *gag*/*pol* LAP cells at a ratio of 10:1 (VSV-G:selection marker) via calcium phosphate precipitation using the profection kit from PROMEGA as recommended by manufacturer. From the time of cotransfection, the cells are kept in non-inducing media. Two days after the cotransfection the cells are selected with histidinol for 10 days until all the non-resistant cells are dead. The cells are then taken off the selection and a limiting dilution is carried out in several 96-well plates such that one third of the wells shows growth. Each clone is split into two tissue culture dishes, one dish is cultured under VSV-G inducing conditions and one dish is cultured under VSV-G repressing conditions. One day later, the induced cells are transfected with the β-gal gene (from pCB/β-gal) via calcium phosphate transfection in order to determine the titering potential of each of the PCL clones. The non-induced cells are transfected with the β-gal gene as well in order to analyze leakiness. of VSV-G expression. Two days after the transfection, the supernatant is harvested and HT1080 target cells transduced with the viral particles carrying the β-gal gene as follows.

On day one, per supernatant three wells of a 6-well plate are seeded with 2x10⁵ HT1080 in 2 ml media (DMEM high glucose with 10% FBS, nonessential amino acids and sodium pyruvate) per well. Cells are incubated exactly for 24 hours at 37°C and 10% CO₂ before transduction the next day.

On day two the media is removed, and one ml media containing 8 µg polybrene/ml is added per well. Cells are incubated for 30 minutes at 37°C and 10% CO₂. Dilute the supernatant by gentle pipetting in media to an approximate concentration of 5 CFU/µl Five, 20 and 50 ml of the diluted supernatant is added into wells 1 to 3, respectively, the media swirled and incubated at 37°C and 10% CO₂ for 24 hours.

On day three one ml of media is added per well and cells incubated at 37°C and 10% CO₂ for 24 hours

On day four an X-gal staining procedure on the transduced cells is carried out

Briefly, the media is drained, and 0.5 ml of fixing solution (PBS with 2% (v/v) formaldehyde and 0.2% (v/v) glutaraldehyde) is added per well of a 12-well plate. The cells are incubated for 5 minutes at room temperature, the fixing solution drained and cells washed with 2 ml PBS per well. The wells are then drained and 0.5 ml of freshly mixed X-gal stain (PBS with 5 mM potassium ferrocyanide, 5 mM potassium ferricyanide, 2 mM MgCl₂, and 1 mg/ml X-gal) is added per well. Cells are incubated from 2 hours to overnight at 37°C and blue cells counted.

Once the titering potential is determined, the four clones with the highest capacity to produce infectious viral particles are kept in culture for several weeks in non-inducing media in order to determine the basal levels of VSV-G expression. Only clones that do not show effects of VSV-G toxicity are useful as stable VSV-G PCLs.

### EXAMPLE 5

### ALTERNATE GLYCOPROTEIN METABOLISM FOR PRODUCTION OF STABLE PSEUDOTYPING CELLS

As noted above, the present invention also provides cell lines which have an altered or mutated glycosylation pathway, which carry or contain an expression vector that directs the expression of an otherwise toxic glycoslylated protein, or fusogenic protein.

For example, Lec 1, 2, and 8 cells (ATCC CRL 1735, 1736, 1737), which are known to have altered glycosylation phenotypes, may be screened by transfection with pMLP-G essentially and selected for antibiotic resistance with a suitable selection marker. Isolated colonies are analyzed by flow cytometry using a monoclonal antibody to VSV-G (*e*.*g*., from Sigma Chemical Corp., St. Louis, MO). Stable cell clones are used for the generation of packaging cell lines.

### EXAMPLE 6

### RABIES G PROTEIN PACKAGING CELL LINES

A plasmid vector containing attenuated rabies virus strain SAD B19 G protein (pSAD61 EMBL/GenBank Acc. No. M31046, Conzelmann, FRCVDA Tubingen, D) is used as a template to PCR amplify the G protein sequence for use in pseudotyping retroviral vectors.

Briefly, the sequence encoding G protein is amplified according to the manufacturer's instructions (Perkin Elmer) utilizing the primers set forth below:

Sequence ID No. 5
5'-3' GTC AAG CTT AAC ATC CCT CAA AAG ACT CAA GGA AAG ATG G

This primer is the sense sequence of the G protein containing the ATG start site, with a *Hind* III site at the 5' end.

Sequence ID No. 6
5'-3' GAT ATC TCG AGC TTA CAG TCT GGT CTC ACC CCC ACT CTT

This primer is the anti-sense sequence of the G protein at the termination of the protein, with a *Xho* I site at the 5i end.
Both the 5' and 3' primers have restriction sites compatible with the *5' Hind* III and 3' *Xho* I sites of the multicloning site of pcDNA3 (Invitrogen SD, CA).

The resultant PCR product is isolated, digested with *Hind* III and *Xho* I and ligated to *Hind* III and *Xho* I digested pcDNA3 to generate the pcDNA3/Rab-G plasmid. Gag/pol pre-packaging lines are transfected with pcDNA3/Rab-G and selected for Neo^{r} resistance. Stable cell lines are selected using the appropriate drug, and the pool subcloned.

### EXAMPLE 7

### ALPHA VIRUS CORE, E1, E2 PACKAGING CELL LINES

The VSV-G protein equivalent in alphaviruses such as the Sindbis virus is a heteroprotein of three proteins: core, E1 and E2. As described in more detail below, a plasmid vector containing Sindbis DNA (ATCC 75891) may be utilized as a template to amplify the core and E1 and E2 glycoproteins for use in pseudotyping retroviral vectors.

Briefly, similar to that described above in Example 6, core, E1 and E2 protein is amplified according to the manufacturer's instructions (Perkin Elmer) utilizing the following primers:

Sequence ID No. 7
5'-3' GTC AAG CTT GCT AGC TAC AAC ACC ACC ACC ATG AAT ACA G

This primer is. the sense sequence of the core protein containing the ATG start site, with a *Hind* III site at the 5' end.

Sequence ID No. 8
5'-3' GAT ATC TCG AGC GGC CGC TCA TCT TCG TGT GCT AGT CAG

This primer is the anti-sense sequence of the E2 protein at the termination of the protein, with a *Xho* I site at the 5í end.

Both the 5' and 3' primers have restriction sites compatible with the 5' *Hin*d III and 3' *Xho* I sites of the multicloning site of pcDNA3 (Invitrogen SD, CA).

The resultant PCR product is isolated, digested with *Hind* III and *Xho* I and ligated to *Hind* III and *Xho* I digested pcDNA3 to generate the pcDNA3/coreE1E2G plasmid. Gag/pol pre-packaging lines are transfected with pcDNA3/coreE1E2G and selected for Neo^{r} resistance. Stable cell lines are selected using the appropriate drug, and the pool subcloned.

### EXAMPLE 8

### SYNTHETIC G PROTEIN PACKAGING CELL LINES

An example of a synthetic G protein is the ectodomain of rabies G, which has been truncated at the anchor residues on the cytoplasmic end of the transmembrane region. This truncation is made using the reagents from Example 5, except that the following 3' primer is utilized:

Sequence ID No. 9
5'-3' GAT ATC TCG AGT TAG ACT CTT CTA CAA CAT GTC ATC AGG

This primer is the anti-sense sequence of the G protein at the anchor region of the protein, with a *Xho* I site at the 5í end.

The resultant PCR product is isolated, digested with *Hind* III and *Xho* I and ligated to *Hind* III and *Xho* I digested pcDNA3 to-generate the pcDNA3/ΔG plasmid. Gag/pol pre-packaging lines are transfected with pcDNA3/ΔG and selected for Neo^{r} resistance. Stable cell lines are selected using the appropriate drug, and the pool subcloned.

### EXAMPLE 9

### Generation of Pre-Producer Cell Lines

### A. Generation of a Pre-Producer Cell Line

Pre-producer cell lines are cells containing *gag*/*pol* genes and a retroviral vector, but lacking an envelope gene. Such cell lines may be produced essentially as follows. Briefly, N2 βgal is used to transduce 293 2-3 cells (which contain only a gag/pol expression cassette), and G418 resistant clones are isolated. Clones are then tested for βgal expression, and a stable positive clone isolated.

### B. Testing Pre-Producer Cell Lines for Growth in Selenium Deficient Media

293 2-3/β gal pre-producer cells are thawed into DMEM-10% FBS and systematically adapted to selenium deficient media, such as DMEM supplemented with insulin/transferrin (IT, Collaborative Research) (see generally, Speier et al., *supra*). This protocol involves initial growth in FBS-containing media and sequential depletion of FBS from the media with concomitant increase in IT-containing media. The adaptation procedure to a selenium-deficient state is usually complete within 2-3 weeks and adapted cells can often be maintained in a selenium-deficient state for more than three months.

### EXAMPLE 10.

### PRODUCTION OF RETROVIRAL VECTOR BY ULTRA-LARGE SCALE TRANSIENT TRANSFECTION

### A. Introduction

High level expression of a newly introduced protein from a cell line is often desired, *i.e*., for expressing gag-pol or an envelope protein in a retroviral vector packaging cell line. However, in some cases cytopathic or cytostatic effects of toxic proteins such as VSV G, can severely limit their maximum stable expression level so as to severely limit or eliminate their utility in a stably transfected and stably expressing cell line. A useful alternative to stable expression is transient expression following transient transfection of an expression plasmid. Such transfections may be accomplished utilizing either DEAE Dextran, electroporation, liposomes, or CaPO₄ precipitation (see *Current Protocols in Molecular Biology or Molecular Cloning*, *a Laboratory Manual* for typical protocols). Following the transient transfection, a percentage of the cells will take up and express reasonably high levels of even a toxic gene for at least a few days. For production of retroviral vectors, transient transfection with CaPO₄ has been the transient transfection method most often chosen for reasons including simplicity, high DNA copy number and expression per cell, and most importantly, high titer vector production. It has been used for small scale vector production with either typical retroviral envelope proteins (Finer et al., *Blood 83*:43-50, 1994; Pear et al., *PNAS USA 90*:8392, 1993) or for pseudotyping with VSV G protein (Burns et al., *PNAS USA 90*:8033-8037, 1993). While this methodology is well adapted to make small or trial quantities of a variety of different vectors, it is much less suitable for medium or larger scale production. For example, CaPO₄ transfection efficiency is known to vary widely depending on exact pH of solutions and a number of poorly understood other parameters. It is generally considered to be intolerant of more than small protocol variations and published protocols (*i*.*e*., *Current Protocols in Molecular Biology or Molecular Cloning, a Laboratory Manual*), specify transfection of cells in 10 cm or smaller plates. Accordingly, published accounts of CaPO₄ transfection typically specify 10 cm or smaller plates for free gas exchange and CO₂ equilibration. Any scale up is then done by working with large numbers of these plates, as for example in the recent paper by J. Yee et al (Yee et al., "A general method for the generation of high-titer, pantropic retroviral vectors: Highly efficient infection of primary hepatocytes," *PNAS USA 91*:9564-9568, 1994). The scale up potential of small plate transfections is clearly limited.

As described in more detail below utilizing culture factories (1200 to 6,000 cm² each) retroviral vector can be produced with crude titers equal or greater than that obtained with transfection in traditional small plates. The protocol is simple, uses common inexpensive reagents to transfect the desired plasmids, and it greatly facilitates the production of much larger quantities of a retroviral vector than are practical with previously reported transient transfection methods. Table one shows the results of one infection of 293 2-3 cells in a 2 layer, 1200 cm² cell factory to produce G-pseudotyped N2 β-gal retroviral vector. The simple protocol described here requires only addition of the CaPO₄ precipitated DNA to the media over the cells, and changing to fresh media after 24 hours, and so is readily scalable to large numbers of cell factories. This makes feasible the production of tens or hundreds of liters of high titer G-pseudotyped vector.

| Time Post Transfection | Titer (BCFU/Ml) | Total Volume Harvested | Total retroviral vector particles |
|---|---|---|---|
| 48 hours | 9.2 x 10⁵ | 200 mls | 1.8 X 10⁸ |
| 72 hours | 1.6 x 10⁶ | 200 mis | 3.2 X 10⁸ |

The cell line 293 2-3 is a MLV gag-pol expressing clone derived from human embryonic kidney 293 cells. The plasmid pMLP-G expresses VSV G from an adenovirus MLP promoter. The plasmid containing the test retroviral vector, pN2 B-gal neo, is co-transfected along with the pMLP-G plasmid. Similar titers may be obtained utilizing either this co-transfection method, or by transfection of pMLP-G alone into a 293 2-3 / B-gal cell line (a 293 2-3 clone, transduced with the B-gal vector).

### B. Production of VSV G-Pseudotyped Retroviral Vector by CaPO4 Bulk Transfection.

### 1. Preparation of cells for transfection.

The 293 cells (ATCC CRL 1573) expressing MLV gag-pol, called 293 2-3 are passed the day prior to the transfection to a 2-layer cell factory (Nunc) of ~1200 cm² cell culture area. This gag pol expressing clone of the cell line 293 is made by standard methods as have been described elsewhere. Between 4 and 48 hours later, preferably 18-24 hours, the cells are used for transfection having reached a level of confluence preferably between 30% and 90%, most preferably 60-80%.

### 2. Preparation of the DNA CaPO₄ precipitate.

Two plasmids are co-transfected in one precipitate. The plasmid MLP-G expresses the VSV G gene, and the plasmid pCBβ-gal contains a retroviral vector expressing β-gal and the neomycin resistance gene. For every 100 cm² of culture area 10-40 µg of each plasmid, preferably ~20 µg, is used. The plasmids are prepared by Quiagen kit standard methods. Optionally they are sterilized prior to transfection by ethanol precipitation or chloroform extraction. For the cell factory ~240 µg of each plasmid in TE (10 mM. Tris, pH 7.4, 1 mM EDTA) are mixed in a 50 ml polypropylene conical tube (Corning or Falcon). and then diluted to 10.8 ml (.9 ml/100 cm²) with water and TE (1/10 TE) such that the final concentration of Tris is 2 mM or less. Then 1.2 mls (.1 ml/100 cm²) of 2.5 M Cacl₂ are mixed in. To a separate 50 ml tube, 12 ml of Hepes buffered saline (2x HeBS) pH ~7.08 is added (see solution recipes below). In a sterile tissue culture hood the 2x HeBS is continually vortexed while the DNA mixture is added by slow dropwise addition from a pipette over a period of ~30 seconds. The resulting precipitate is allowed to sit at room temperature for 15 minutes.

### 3. Transfection.

The standard growth media is removed from the cells, the DNA precipitate is added to it, and the media plus DNA is gently replaced into the cell factory. After 4 to 24 hours, preferably 16-24 hours, the media is removed and the cells are rinsed 2x with ~50 ml of PBS. Fresh media is then replaced at daily interval for as many days of harvesting as desired but at least two days, day 2 and day 3 post transfection are always collected as the typically optimal days for best titer.

### 4. Solutions

2.5 M CaCl₂: 367.4 g CaCl₂ dihydride per liter, filter sterilized.
2x HeBS: 0.21 g NA₂HPO_{4,} 16.4 g NaCl, 11.9 g HEPES acid, ~800 ml H₂O

Adjust pH to 7.08 with 10 N NaOH, and H₂O to 1 liter. Filter sterilize, and store at -20°C in aliquots.

### EXAMPLE 11.

### A VSV-G FUSION PROTEIN WHICH PREVENTS INDUCTION OF SYNCYTIA IN PRODUCER CELLS

### A. Background

As discussed in more detail below, the last 49 amino acids of the C terminus of VSV-G, which includes the transmembrane domain (TM), can be replaced with the last 54 amino acids of the MoMLV env. This chimeric protein includes several MLV components: the trans membrane domain, the amphipathic loop and the R peptide.

Other chimeric proteins that can similarly be made include fusions of the two envelope proteins within or outside the TM domains, *e.g.*, the entire or part of the VSV-G TM domain is fused to MLV sequences within the C terminus. As utilized herein, it should be understood that envelope proteins and sequence combinations derived from fusion of the two protein sequences will lead to attenuated fusogenicity of the resulting chimera.

### B. Construction of a VSV-G Fusion Protein

As an example the following describes the replacement of the last 49 amino acids of the C-terminus of VSV-G with the equivalent C-terminus 54 amino acids of MLV.

The resulting amino acid sequence is shown below. The junction is denoted (::):
(VSV-G Indiana) ...GWFSSWK:: IMGPLIVLLLFGP... - (Ampho env-4070A)...

### 1. Materials:

MoMLV 4070A Ampho envelope expression vector pEnvAm-Dra which includes the SV40 poly A. The VSV (Indiana strain) envelope protein G is derived from pMLP-G. pSC6 is a CMV promoter expression cassette (see WO 91/02805).

The construction of the chimeric protein utilizes the overlapping PCR method, and the following primers:

Primer I: (VSV-G: 803 - 825 bp; including a Pst I site) (Sequence ID No. 10)
PstI
5' GG ATC TCT TTG CTG CAG CCA GAT

Primer II: (Junction sequence:1398-1415 of G and 7616-7634 of Ampho) (Sequence ID
No. 11)
5' TGG TTC AGT AGT TGG AAA / ATC ATG GGA CCT CTA ATA G

Primer III: (Complimentary to primer II) (Sequence ID No. 12)
5' C TAT TAG AGG TCC CAT GAT / TTT CCA ACT ACT GAA CCA

Primer IV: (SV40 poly A from pEnvAm-Dra; 161-185 bp; Bam HI site; negative strand)
(Sequence ID No. 13)
BamHI
5' C ATG TCT GGA TCC CCA GGA AGC TCC

### 2. Construction of the expression vector:

PCR amplified DNAs using the two sets of primers (I plus II and III plus IV) are annealed and PCR amplified using primers I and IV. The resulting DNA is digested with the restriction enzymes *Pst* I and *Bam* HI. The resulting fragment of about 900 bp of hybrid G/A is cloned into pSC6 (*Eco*RV and *Bam*HI digested) together with the *Cla* I (blunted) to *Pst* I (800 bp) of VSV-G DNA. This plasmid is designated pG/A.

### 3. Cell culture/titer assay:

(a) The hybrid env plasmid DNA (pG/A) is transfected using the CaPO₄ precipitate method into the gag-pol expressing 293/2-3 cells as described above. Formation of syncytia is assessed visually after 1, 2 and 3 days.
(b) In parallel, pG/A is cotransfected with pKT-1 into 293/2-3 and SCV21 or DA gag-pol cells to assess transient titers and envelope functionality. This transient (48 hours) expression method is described above. The cotransfection of pMLP-G plus N2 βgal into the above-mentioned cells is used as a control in this experiment.
(c) In order to assess stable expression of the hybrid envelope in producer cells: forty eight hours post transfection the cells (from (b) above) are subjected to G418 selection for 10 days and stable pools are tested for vector production, infectivity and any tendency to form syncytia.

### EXAMPLE 12.

### USE OF RECOMBINANT CYTOMEGALOVIRUS EXPRESSING VESICULAR STOMATITIS VIRUS-G PROTEIN TO PSEUDOTYPE RETROVIRAL VECTORS

### A. Materials and Methods

### 1. Viruses and cell lines.

Wild type MCMV (Smith strain K181+), RM427, and RMCMVG are grown and titered on NIH3T3 cells as described in Manning et al., *J*. *Vir*. *66*:3794-3802, 1992; Stoddart, *J*. *Vir. 68*(10):6243-6253, 1994. NIH3T3, HeLa, 293, and 293 gag /pol(a 293 line containing *gag* and *pol* but lacking an *env* gene) cells are grown in Dulbecco's modified Eagle's medium supplemented with 10% fetal calf serum.

### 2. Plasmids and preparation of recombinant virus.

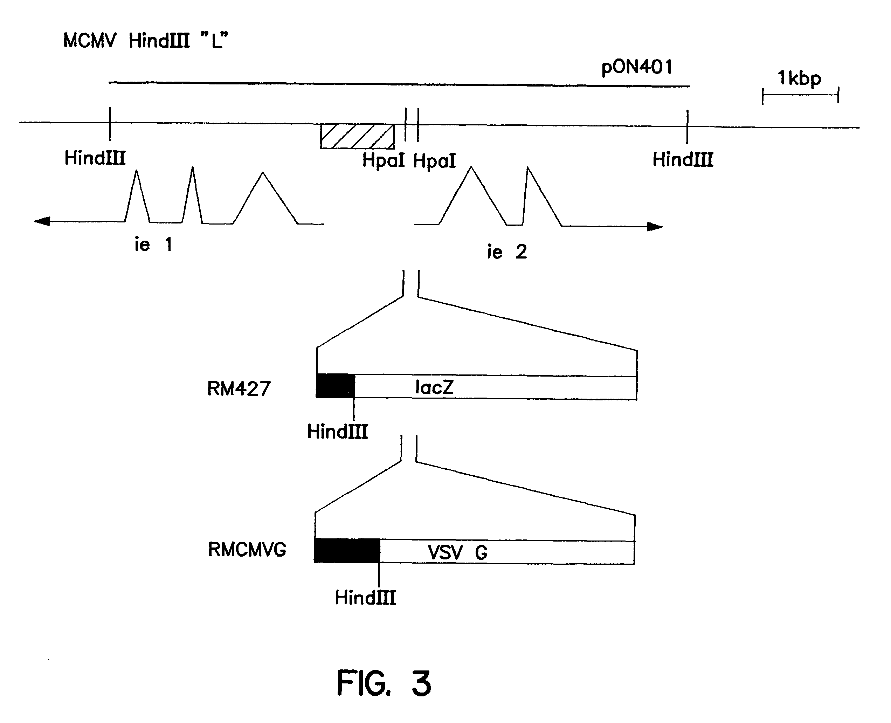

Plasmid cloning is by standard technique (Sambrook et al, *Molecular Cloning: A Laboratory Manual*, 2d ed., Cold Spring Harbor Laboratory Press, 1989). pON401 contains the Hind III L fragment of MCMV (Manning et al., *Vir*. *66*:3794-3802, 1992). pCMV-G contains the VSV-G protein under transcriptional control of the HCMV *ie* 1 promoter-enhancer. To construct pON401CMVG, the 2665 -bp *Hinc* II fragment of pCMV-G containing VSV-G is cloned between the *Hpa* I sites of pON401 (Figure 3). This results in a deletion of the 79-bp Hpa I fragment in pON401. pMLP-G contains the VSVG protein under transcriptional control of the adenovirus major late promoter.

Protocols for the production and isolation of recombinant MCMV have been described (Manning et al., *Vir*. *167*:477-184, 1988). Briefly, 15 µg of linearized pON401CMVG is co-transfected with RM427 viral DNA using calcium phosphate precipitation. The amount of RM427 DNA used in the transfection is determined empirically for each viral DNA preparation. The co-transfections are harvested when cytopathic effect (cpe) is complete, approximately 5 days post transfection.

To isolate *β-gal-* recombinants, a limiting dilution assay is done. Briefly, 96-well tissue culture plates are seeded with NIH3T3 cells (10⁴ cells/well). Recombinant virus pools arising from the co-transfection are titered, and then used to infect the 96-well plates at a dilution which would contain approximately 75 plaque forming units (pfu) per 96 well-plate. When cytopathic effect (cpe) is evident (about 7 days after infection) methyl umberryliferone (MUG) is added to all wells at a final concentration of 150 µg/ml. At various time points (3-24 h) the plate is put on a UV light box and photographed. Wells containing parental *β-gal+* virus (RM427) would fluoresce. Uninfected wells, or wells infected by a β-*gal*- recombinant, would not fluoresce. Virus from wells with cpe and no fluorescence is grown up and analyzed by DNA blot analysis.

### 3. DNA analysis.

Viral DNA is prepared from purified virions (Vieira et al., *J*. *Virol*. *68*:4837-4846, 1994) and subjected to DNA blot analysis as previously described (Manning et al., *Vir. 167*:477-484, 1988). -

### 4. Isolation of infected-cell proteins and immunoblotting.

To prepare total cell protein, cells are infected with virus at a multiplicity of infection (MOI) of 10. At the appropriate time point, cells are rinsed with phosphate buffered saline and then lysed in NP40 lysis buffer (100 mM NaCl, 20 mM Tris pH 7.5, 1 mM EDTA, 0.5% NP40, 0.5% deoxycholate) supplemented with aprotinin (2 µg/ml), pepstatin (1 µg/ml), leupeptin (2 µg-ml), and Peflabloc SC (1 mg/ml). All protease inhibitors are purchased from Boehringer Mannheim. Plates are incubated on ice for 5 minutes. Cells are harvested by scraping, placed in 1.5 ml microcentrifuge tubes, and spun in a microfuge at high speed for 10 minutes at 4°C. Supernatants are collected and stored at -80°C until analyzed.

Infected-cell proteins are separated on 10% SDS-polyacrylamide gels (Novex), transferred to nitrocellulose, and subjected to Western blot analysis. The primary antibody is a mouse monoclonal to VSV-G glycoprotein (Sigma ImmunoChemicals) used at a dilution of 1:100,000. Peroxidase-conjugated goat anti-mouse (Boehringer Mannheim) at a dilution of 1:30,000) is used as the secondary antibody. Blots are developed using ECL (Amersham) following manufacturer's directions.

### 5. Producer cells, retroviral vector production, and titration.

To make the cell line for use in the production of pseudotyped vector, 293 gag /pol cells are infected with N2 β-gal, a VSV-G pseudotyped retroviral vector. N2 βgal contains the genes for neomycin phosphotransferase (neor) and (3-galactosidase (β-gal). G418 resistant colonies are selected. Staining of the resulting cell line with Bluogal (GIBCO BRL) shows that all cells are blue. To produce VSV-G pseudotyped retrovirus, 10 cm plates of 293 gag /pol (N2 β-gal) cells are infected with RMCMVG at the desired MOI in a total volume of 2 ml. Two hours after infection, the cells are washed twice, and then incubated in 2 ml medium until harvested. Twenty four or 48 hours after infection, supernatants are filtered (0.45um pore) and stored at -80°C until titered. Supernatants are titered on HeLa cells. The day before infection 10 cm plates are seeded with 2 X 10⁶ cells. On day 1, plates are infected with 100µl of supernatant in 2 ml of media containing Polybrene (Sigma; 8 µg/ml). After a 2 hour incubation, the plates are washed, and incubated overnight with fresh media. On day 2, plates infected with supernatants from RMCMVG-infected cells are split 1:10 ant 1:100. Those from mock or wt MCMV-infected supernatants are split 1:2. On day three, G418 (Sigma; 1 mg/ml) is added to the media. Ten to fourteen days later, colonies are stained and counted.

### B. Results

### 1. Construction of RMCMVG.

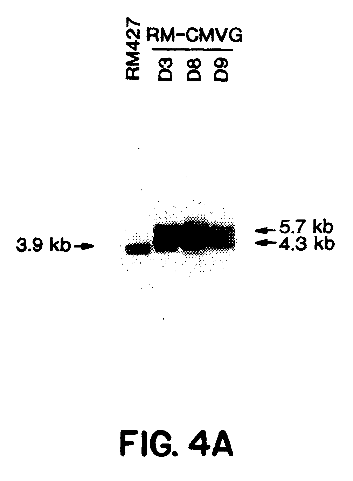

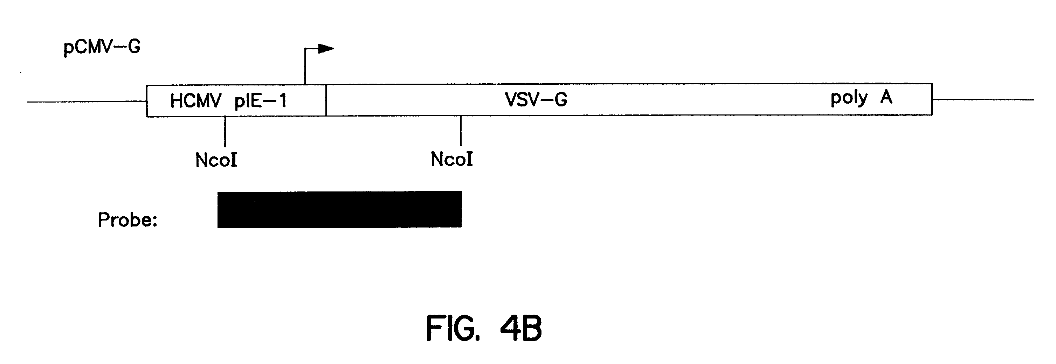

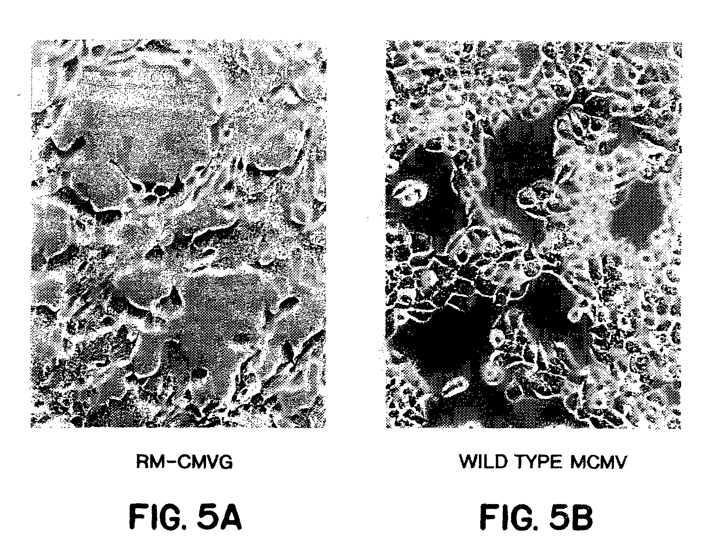

RMCMVG is isolated following co-transfection. of NIH3T3 cells with RM427 DNA and pON401-CMVG (Fig 3). After screening eight 96-well plates for *β-gal*-recombinants, six wells are identified that contained β-*gal* virus. Three of these are grown up and plaque purified an additional time. These represent recombinants arising from two independent co-transfections. Viral DNA is prepared from these three isolates and subjected to DNA blot analysis. As shown in Figure 4, all three recombinant viruses contain the 5.7 kbp and 4.3 kbp HindIII bands predicted for a VSV-G recombinant. Isolate D9 is grown up and stock titers of approximately 1 x 10⁸ plaque forming units (PFU)/ml are obtained. This is identical to stock titers of wild type MCMV indicating that VSV-G expression is not toxic to MCMV replication in cell culture. In contrast to wild type MCMV, RMCMVG plaques demonstrated a marked syncytium phenotype (Figure 5A).

### 2. RMCMVG expresses VSV G glycoprotein in permissive and non-permissive cells.

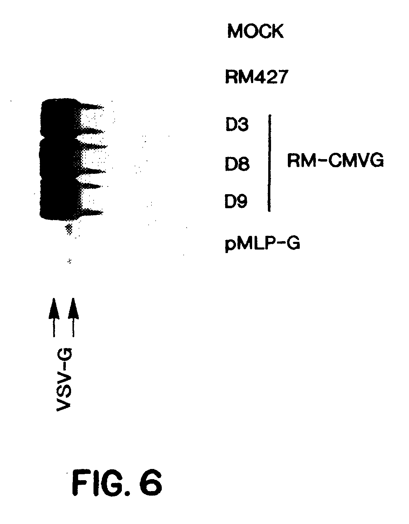

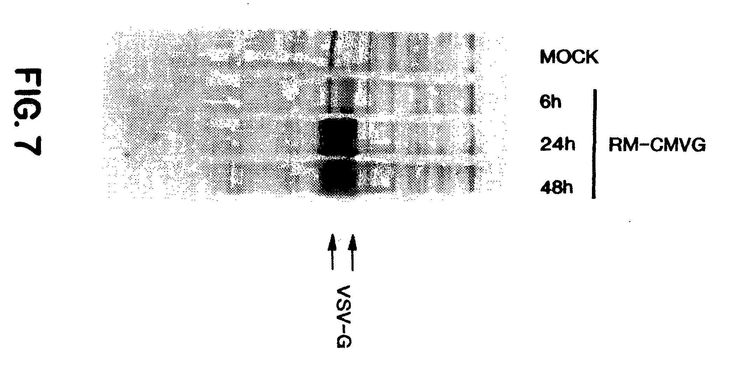

NIH3T3 cells are infected with the three isolates of RMCMVG and subjected to Western blot analysis. As demonstrated in Figure 6, NIH 3T3 cells infected with all three isolates expressed abundant amounts of G protein at 24 hours after infection. Cells infected with RM427 do not show any VSV-G expression. As a positive control, a lane containing G glycoprotein produced after transfection of 293 cells with pMLP-G is included. To determine whether RMCMVG expresses G protein in nonpermissive cells, 293 cells are infected with RMCMVG and protein is harvested at 6, 24, and 48 hour post infection. As shown in Figure 7, VSV-G protein expression is detected as early as 6 hour post infection, but peaked at 24 hour post infection.

### 3. RMCMVG infection can be used to pseudotype retroviral vectors.

To investigate the ability of RMCMVG to pseudotype retroviral vectors, 293 gag /pol (N2 β-gal) cells are infected with either wild type MCMV or RMCMVG. Supernatants are collected from cells at 24 hour and 48 hour after infection and titered. As shown in Table 1, peak neo titers of approximately 1 x 10⁴ colony forming units (CFU)/ml are found in the supernatants of cells 24 hour after infection by RMCMVG at a MOI of 10 or 20. No retroviral titers are found in the supernatants of mock infected cells or cells infected with wild type MCMV.

**Table 1**

| VSV G-pseudotyped vector production from 293 gag /pol (N2 β-gal) cells following infection with RMCMVG or wild type MCMV | | | |
|---|---|---|---|
| | | Titer (CFU / ml)^{a} | |
| Virus | MOI^{b} | 24 h | 48 h |
| RMCMVG | 1 | 9.0 x 10³ | 2.0 x 10² |
| | 10 | 2.3 x 10⁴ | 4.0 x 10³ |
| | 20 | 4.2 x 10⁴ | 1.2 x 10⁴ |
| | | | |
| wt MCMV | 1 | nd^{c} | nd |
| | 10 | nd | nd |
| | 20 | nd | nd |
| | | | |
| Mock | | nd | nd |

Since 293 cells are not permissive for MCMV replication there should be no RMCMVG in the supernatants. When supernatants are titered for RMCMVG, titers of 200 plaque forming units (PFU) /ml are found (data not shown). This small amount of MCMV is probably virus remaining from the initial infection. The large size of MCMV (300 nm) relative to retroviral vector should allow for simple removal of this residual MCMV from the supernatant.

## Claims

1. A retroviral packaging cell line comprising a human host cell, containing a gag/pol expression cassette and an expression vector, wherein said expression vector comprises a murine cytomegalovirus immediate early (CMV IE) promoter operably linked to a nucleic acid sequence encoding a toxic or fusogenic protein.

2. The packaging cell line according to claim 1 wherein said fusogenic protein is Vesicular Stomatitis Virus-G protein.

3. A retroviral producer cell line, comprising a retroviral packaging cell line according to claim 1 or claim 2, said retroviral packaging cell line additionally including a retroviral vector construct.

4. A method for producing retroviral particles, comprising providing a murine cytomegalovirus in a retroviral producer cell line according to claim 3.

## Patentansprüche

1. Retrovirale Verpackungszelllinie, umfassend eine humane Wirtszelle, die eine gag-pol-Expressionscassette und einen Expressionsvektor enthält, wobei der Expressionsvektor einen murinen Cytomegalovirus-immediate early (CMV IE)-Promotor funktionell an eine Nucleinsäuresequenz, die für ein toxisches oder fusogenes Protein codiert, gebunden umfasst.

2. Verpackungszelllinie nach Anspruch 1, wobei das fusogene Protein vesikuläres Stomatitis-Virus-G-Protein ist.

3. Retrovirale Producer-Zelllinie, die eine retrovirale Verpackungszelllinie nach Anspruch 1 oder Anspruch 2 umfasst, wobei die retrovirale Verpackungszelllinie zusätzlich ein retrovirales Vektorkonstrukt enthält.

4. Verfahren zur Herstellung retroviraler Partikel, umfassend Bereitstellen eines murinen Cytomegalovirus in einer retroviralen Producer-Zelllinie nach Anspruch 3.

## Revendications

1. Lignée cellulaire d'empaquetage de rétrovirus comprenant une cellule hôte humaine, contenant une cassette d'expression gag/pol et un vecteur d'expression, dans laquelle ledit vecteur d'expression comprend un promoteur précoce immédiat de cytomégalovirus murin (CMV IE) lié de façon opérationnelle à une séquence d'acide nucléique codant une protéine toxique ou fusogène.

2. Lignée cellulaire d'empaquetage selon la revendication 1, dans laquelle ladite protéine fusogène est la protéine G du virus de la stomatite vésiculaire.

3. Lignée cellulaire productrice de rétrovirus, comprenant une lignée cellulaire d'empaquetage de rétrovirus selon la revendication 1 ou la revendication 2, ladite lignée cellulaire d'empaquetage de rétrovirus comprenant en plus une construction de vecteur rétroviral.

4. Méthode de production de particules rétrovirales, comprenant le fait de fournir un cytomégalovirus murin dans une lignée cellulaire productrice de rétrovirus selon la revendication 3.
